# EUROPEAN PATENT SPECIFICATION

(11) **EP 0 519 971 B1**
(45) Date of publication and mention of the grant of the patent: **15.01.1997**
(21) Application number: 91905859.4
(22) Date of filing: 13.03.1991
(51) Int. Cl.: C07D 487/04, A61K 31/505

(54) **NOVEL PHARMACEUTICALLY ACTIVE COMPOUNDS WHICH CAN BE FORMED FROM 1,3,4,6,7,8-HEXAHYDRO-2H-PYRIMIDO-(1,2-a)-PYRIMIDINE**
NEUE, VON 1,3,4,6,7,8-HEXAHYDRO-2H-PYRIMIDO-(1,2-A)PYRIMIDIN AUSGEHENDE PHARMAZEUTISCH AKTIVE VERBINDUNGEN
NOUVEAUX COMPOSES PHARMACEUTIQUEMENT ACTIFS, POUVANT TRE PRODUITS A PARTIR DE LA 1,3,4,6,7,8-HEXAHYDRO-2H-PYRIMIDO-(1,2-a) PYRIMIDINE

(30) Priority: 13.03.1990 FI 901239
(43) Date of publication of application: 30.12.1992
(73) Proprietor: INSTITUT FÜR SOZIAL-MEDIZINISCHE FORSCHUNG SA, CH-2015 Areuse (CH)
(72) Inventor: DAHLGREN, Atti-La, S-181 37 Lidingö (SE); DAHLGREN, Ake, CH-2015 Areuse (CH)
(74) Representative: Fogelberg, Lennart
(86) International application number: SE9100192
(87) International publication number: WO9113886

## Description

The present invention relates to novel compounds which can be formed from 1,3,4,6,7,8-hexahydro-2H-pyrimido-(1,2-a)-pyrimidine (HHPP), said novel compounds of the formula 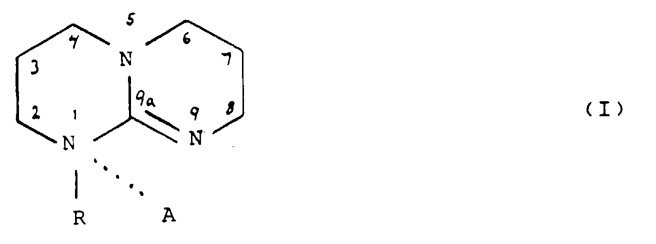 wherein R is hydrogen or an alkyl group, preferably -CH₃, and wherein the dotted line represents a salt linkage or, when R is hydrogen, such hydrogen being replaced thus that a covalent bond is formed between 1,3,4,6,7,8-hexahydro-2H-pyrimido-(1,2-a)-pyrimidine and the group A, and wherein A is a residue of an acid or acid anhydride of classes comprising specific acids and acid anhydrides identified below. Further, the man skilled in the art will recognise when an acid may be replaced with its anhydride, and vice versa, and such replacement is within the scope of the invention. The invention relates particularly to such novel compounds having pharmaceutical activity and to preparations including such novel compounds.

In accordance with a first aspect of the present invention, there is provided a pharmaceutically active compound of formula I wherein the group A is a residue of an acid HA or H₂A selected from indole carboxylic acid, preferably from 2-indole carboxylic acid, 2,4,6-trihydroxy benzaldehyde being made subject to oxidation, 3-nitro-o-toluic acid, phthalic anhydride and benzoic anhydride, or an ester or acid chloride thereof; the acid being further selected from 4-chloro-2-nitrobenzoic acid, 4-nitrophenylacetic acid, 2,4-dinitrophenylacetic acid, 5-chloro-2-nitrobenzoic acid, 4-chloro-3-nitrobenzoic acid, 5-nitrosalicylic acid, (2-hydroxy-5-nitrobenzoic acid), 3-hydroxy-4-nitrobenzoic acid, 2-iodobenzoic acid, 5-amino-5-hydroxybenzoic acid, 5-nitroisophthalic acid, (5-nitrobenzene-1,3-dicarboxylic acid), an amino acid, such as a natural amino acid, such as selected from glycine, L-glutamine and L-histidine, and further such as an amino acid selected from L-valine, L-leucine, and 4-aminobutyric acid (GABA), L-glutamic acid, L-serine, L-lysine, L-proline, L-alanine, L-cysteine, L-4-hydroxyvaline, L-aspartic acid, L-phenylalanine, L-tryptophane, and L-methionine.; or a neutral amine salt of such a pharmaceutically active compound. In a further embodiment of the invention the acid giving the acid residue A is boric acid. The salt formed of HHPP and boric acid, or a derivative thereof, may advantageously be employed in solution with a sugar.

Such a pharmaceutically active compound may be (a) a salt formed between the compound of the formula I, and a carboxyl group derived from a second compound HA, when the dotted line is a salt linkage, (the chemist of ordinary skill will realize that such linkage is not exclusively to the 1 nitrogen atom, as will be further discussed below, and the illustration of the salt link to the 1 nitrogen atom is not intended to limit the invention in such respect) or (b) an amide defined by the 1-amino group of the compound of the formula (I) and a carboxyl or carbonyl group of the second compound HA.

An embodiment of the first aspect of the present invention resides in a compound having any one of the formulae (II) to (VII) hereinafter. A further embodiment is the compounds disclosed by other Examples.

In accordance with the second aspect of the present invention, there is provided a method of preparing a compound in accordance with the first aspect of the invention, comprising reacting HHPP with one of said second compounds, and optionally neutralising the resultant product. The reaction can be carried out at room temperature, although slight heating may be beneficial. The reaction is usually performed in a solvent or mixture of solvents.

In the case where the target compound is the salt (a) above, then the reaction is quantitative and it is preferred to effect the reaction using equivalent amounts of the starting compounds. To avoid hydrolysis of the salt, it is preferred to perform the reaction in a nonaqueous solvent or solvent mixture in which the starting materials are soluble but the desired final product is insoluble, eg a non-polar solvent or mixture such as chloroform and diethylether. The product then precipitates out and can be filtered and dried under vacuum.

HHPP is soluble in water, chloroform, ethanol and methanol, but not e.g. acetonitrilic or ethyl acetate. 2-Indole carboxylic acid is soluble in ether and protic solvents (e.g. ethanol, methanol etc.). The compound of the formula (II) hereinafter is soluble in water, ethanol and acetonitrile, but not in non-polar solvents.

In the case where the target compound is the amide (b) above, the reaction may be effected in any suitable known way, for example, by reaction may be effected in any suitable known way, for example, by reaction between the compound of formula (I) and the second compound, if necessary after the latter has been esterified. In the case where the second compound is phthalic anhydride or benzoic anhydride, the reaction can be effected directly in the presence of a dilute mineral acid. Where appropriate, other reactive groups on either of the starting compounds may be blocked before amide synthesis, followed by unblocking.

With regard to the optional neutralising step, it will be appreciated that the compounds of the present invention have more than just the nitrogen atom at the 1-position of the pyrimidino-pyrimidine ring and so may exhibit a certain basicity. Neutralisation can be effected using an acid, such as dry HCl gas which may be bubbled through a solution of the compound to be neutralised in a dry solvent, eg dry ethyl acetate, followed by evaporation and vacuum drying.

Drying of the solvents and the HCl used for neutralisation can be effected using any of the known techniques. Solvents (chloroform, methanol or ethyl acetate) can be dried with calcium hydride or using a molecular sieve (3-4A) and distilled prior to use. Hydrogen chloride can be synthesized from sodium chloride and concentrated sulphuric acid or can be taken from a gas cylinder. To ensure the dryness of HCl, it can be bubbled through concentrated sulphuric acid.

The invention also provides a pharmaceutical preparation comprising a pharmaceutically active compound according to said first aspect, or when prepared by the method of said second aspect, in a pharmaceutically acceptable carrier. Preferably, the carrier is one which allows topical application of the medicament, eg a cream.

The pharmaceutical properties of the compounds allow their therapeutic use in the treatment of viral diseases such as herpes or human papilloma virus (genital warts). They may further be used in the combat of human immunodeficiency virus and cancer. Further they may be used as a muscle relaxant. Thus, the present invention also resides in the use of a compound according to said first aspect, or a compound prepared by a method according to said second aspect, in the manufacture of a medicament for the treatment of viral diseases.

HHPP is a known compound which is available from Fluka Chemie AG of Buchs, Switzerland and which also known as 1,5,7-triazabicyclo[4.4.0]dec-5-ene (TBD).

The present invention will now be described by way of the following non-limiting examples:

### Example 1

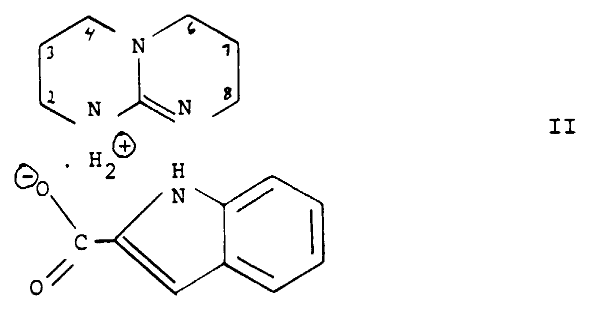

1.000 g (6.2 mmol) of 2-indole carboxylic acid (MW 161.16) is dissolved in 40 ml of dry diethylether. 0.864 g (6.2 mmol) of HHPP (MW 139.19) is dissolved in 30 ml of dry chloroform and is then slowly added dropwise to the first mixture at room temperature. The mixture is stirred for 1 hour. A white precipitate develops which is filtered and then washed with a small amount of other/chloroform-mixture. The product is dried under high vacuum. The yield of the product, which is a salt, is 1.818 g (97% yield).

The purity of the salt is confirmed by TLC-chromatography (Merck DC-Fertigplatten Kieselgel 60 F₂₈₄, elution with dry acetonitrile). No traces of the starting materials are observed on the TLC-plate. The immovable spot of the salt is visible in UV-light at 282 nm and gives a strong, brown colour reaction with Dragendorf-reagent.

The product of the reaction between HHPP and 2-indole carboxylic acid as described in Example 1 above has been investigated and has the formula (II) above.

From elemental analysis, the compound was determined to have an empirical formula of C₁₆H₂₀N₄O₂ and a molecular weight of 300.3634. It is proposed on the basis of the elemental analysis, IR spectroscopy [base peaks; =N-H 3415 at 3470 cm⁻¹: product peak; (=NH₂)⁺ 3215 cm⁻¹), and carbon-NMR spectroscopy [with respect to the acid and base spectra, the peaks of the compound do not show significant shifts, except in the case of the two peaks corresponding to the carbon atoms of the C.COOH fragment which are shifted as expected: acid peaks, shift (δ) 165.14 at 286.12 ppm: product peaks, shift (δ) 152.43 at 170.20 ppm]. This formula is also supported by information gained from mass-, hydrogen-NMR-, ultraviolet- and visible light-spectroscopy.

### Example 2

The salt of 1,5,7-triazabicyclo-[4.4.7]dec-5-ene (TBD, HHPP) with indole-2-carboxylic acid is prepared by mixing in equimolar proportion, the carboxylic acid dissolved in acetone and TBD dissolved in ethanol. The product immediately precipitates and has a m.p of 236-238°C. It can be recrystallized from ethanol which raises the m.p to 242-245°C. Recrystallization from water is also possible. Melting is not accompanied by decomposition. Chloroform or other chlorinated solvents must not be used to dissolve TBD base, owing to reaction of the letter with the solvent.
Structure: A 1H-NMR spectrum of the present salt corroborated the 1:1 molar proportion of the two components of the salt. Elemental analysis gave the expected value for carbon, hydrogen and nitrogen. Solubility: The salt is moderately soluble in water giving a solution of pH 6.5. Solubility in distilled water at room temperature is 70 mg/ml in physiological (0.9%) sodium chloride solution 50 mg/ml. Solubility in absolute ethanol is about 160 mg/ml.

### Example 3

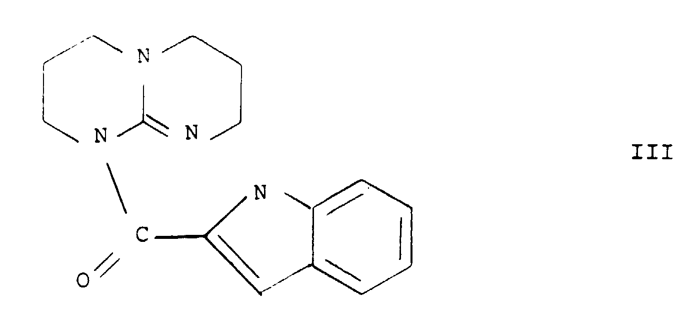

In a variation of the method of Example 1, equimolar quantities of HHPP and 2-indole carbonyl chloride may be reacted together in solvent form and in the presence of a suitable catalyst to produce an amide having the formula (III).

### Example 4

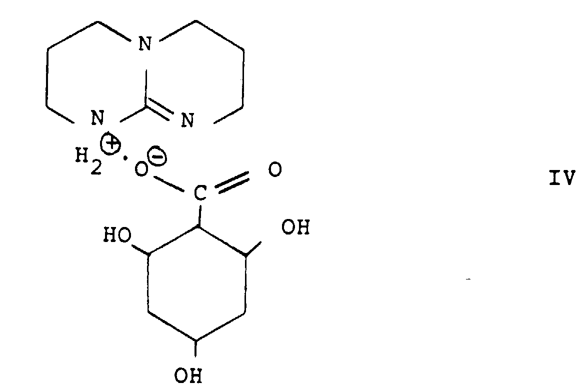

Equimolar quantities of HHPP and 2,4,6-trihydroxybenzaldehyde are dissolved in 3 ml of isopropanol at room temperature. The temperature rises to about 35°C and results in a clear red liquid which yields a viscous red liquid on evaporation. The liquid is alkaline, is soluble in water and ethanol and can be neutralised if required.

### Example 5

0.6 gram of HHPP is dissolved in 2 ml of chloroform at room temperature (22°C). 0.6 gram of 3-nitro-o-toluic acid is likewise dissolved in 2 ml of chloroform at room temperature and the solution of HHPP is added dropwise thereto whilst stirring. The exothermic nature of the reaction causes a temperature rise to about 50°C. The reaction product is a clear yellow liquid which yields a yellow crystalline product on evaporation. The crystalline product is soluble in water, ethanol and chloroform and has a neutral pH.

An amide formed from the starting materials has the formula 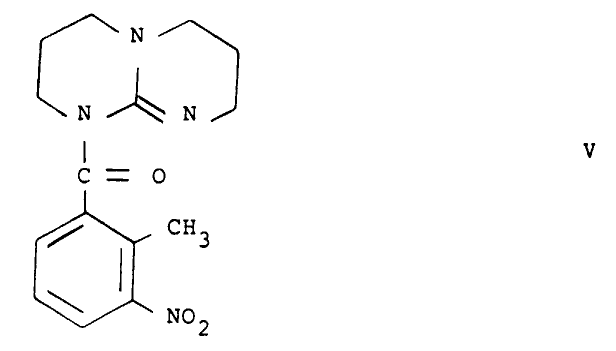 however, proof of amide formation has not yet been secured.

### Example 6

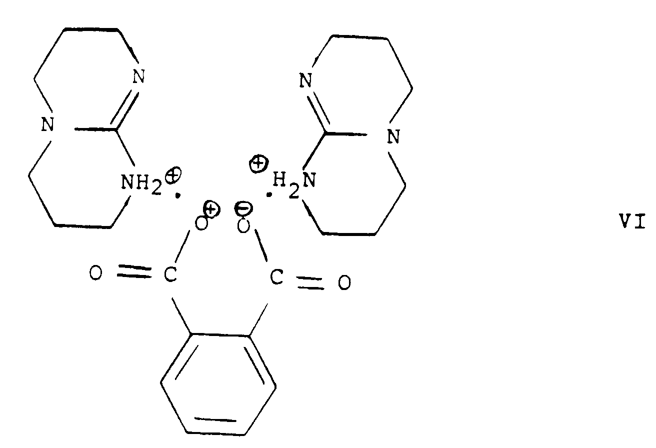

0.6 gram of HHPP is dissolved in 2 ml of isopropanol at room temperature. 0.6 gram of phthalic anhydride is dissolved in 2 ml of hot (50°C) isopropanol. The solution of HHPP is added dropwise to the anhydride, the temperature finally rising to 45°C and resulting in the formation of a clear liquid product which yields a white crystalline product on evaporation. The crystalline product of formula VI is soluble in water and ethanol and has a pH of 8.5 but can be neutralised if necessary.

### Example 7a

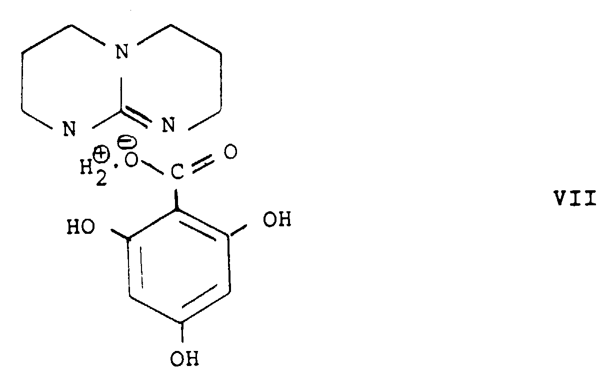

1.2 grams of HHPP is mixed dry with 0.6 gram of benzoic anhydride and 3 ml of chloroform are added to the mixture at room temperature with stirring. The temperature rises to about 35°C and the product is a clear liquid of pH 8.5. The liquid product is neutralised with a 10% solution of ortho-phosphoric acid in ethanol. The neutralised liquid is evaporated to yield a white crystalline product which is soluble in water and ethanol.

### Example 7b

Example 7a is repeated except that the liquid product is neutralised with 15% aqueous hydrochloric acid.

Evaporation of the neutral liquid yields a clear viscous liquid which is soluble in water and ethanol.

### Examples 8 -17: TBD salts of strong aromatic carboxylic acids

The following salts of TBD have been prepared from commercially available aromatic acids manufactured by FLUKA:

| No. | Structure | M.p.,°C | Solubility in water (g/100ml) |
|---|---|---|---|
| Example 8 | TBD 4-chloro-2-nitrobenzoate | 190-193 | 10 |
| Example 9 | TBD 4-nitrophenylacetate | 103-106 | 20 |
| Example 10 | TBD 2,4-dinitrophenylacetate | undefined | 30 |
| Example 11 | TBD 5-chloro-2-nitrobenzoate | 144-148 | 40 |
| Example 12 | TBD 4-chloro-3-nitrobenzoate | 174-176 | 40 |
| Example 13 | TBD 5-nitrosalicylate (2-hydroxy-5-nitrobenzoate) | 200-202 | 160 |
| Example 14 | TBD 3-hydroxy-4-nitrobenzoate | 173-175 | 30 |
| Example 15 | TBD 2-iodobenzoate | 142-144 | 10 |
| Example 16 | TBD 5-amino-5-hydroxybenzoate | undefined | 10 |
| Example 17 | TBD 5-nitroisophthalate (5-nitrobenzene-1,3-dicarboxylic acid bis TBD salt) | undefined | 30 |

The melting points given in Examples 8-17 refer to recrystallized samples. Solubilities were determined with the crude samples and indicate the volume of water sufficient to dissolve one gram of the salt.

The general procedure followed was to dissolve the 5.5 mmol of the acid component in acetone and combine this with a solution of 5 mmol of TBD. If the product did not crystallize directly from the reaction mixture, the solution was evaporated and the residue treated with ether. A sample was recrystallized from ethanol to obtain the final melting point (see above).

Information specific for the individual compounds is given in the following:

### TBD 4-chloro-2-nitrobenzoate (Example 8)

Evaporation of the solution and trituration of the residue with ether gave 1.15 g (68%) of the crude salt mp. 182-188°C.

### TBD 4-nitrophenylacetate (Example 9)

Unlike the other salts this was prepared by stirring a suspension of the acid in ether with TBD added as a solid. Yield of crude product 1.6 g (100%), m.p. 177-188°C.

### TBD 2.4-dinitrophenylacetate (Example 10)

The dark violet product (1.14 g, 62%) precipitated directly from the acetone solution, m.p. undefined. It seems to decompose on recrystallization.

### TBD 5-chloro-2-nitrobenzoate (Example 11)

On evaporation of the acetone solution a syrup was obtained which was crystallized from ethyl acetate containing about 10% of ethanol. Yield 0.89 g (52%).

### The structure of the TBD salt of 5-chloro-2-nitrobenzoic acid (Example 11)

In view of the simplicity of its preparation the only alternative to the formulation of the product as a salt is that it is an amide. The latter possibility can however be excluded based on the following evidences.

### 1) Chemical reasoning

TBD is a very strong base and 5-chloro-2-nitrobenzoic acid is a relatively strong acid and therefore it is highly probable that the two should form a stable salt. Amide formation at room temperature is highly improbable. This would require either the use of an acid chloride or the application of prolonged heating. The salt was however prepared at room temperature.

### 2.) Elemental analysis

Calculated for the molecular formula of the salt (C₁₄H₁₇C₁N₄O₄): C 49.3%, H 5.0%, N 16.4%. Found: C 49.2%, H 5.1%, N 15.7%. The amide (C₁₄H₁₅C₁N₄O₃) would have required: C 52.1%, H 4.7%,
N 17.4%.

### 3) Infrared spectrum

The infrared spectrum of the salt was in conformity with the supposed structure but did not provide additional proof since unfortunately wavenumbers expected for the carbonyl bend of an amide and that for an acid fall into the same range.

4) Proton and carbon-13 NMR spectra at 100 MHz Both proton and carbon-13 spectra of TBD 5-chloro-2-nitrobenzoate salt confirm the proposed structure. The most important feature of the proton spectrum is a broad NH signal at 9.6 ppm integrating for two protons. Also intensity ratio of the well resolved signals of the benzoate part and of the methylene protons of the TBD part is as expected i.e. 1:4. Proton NMR of TBD base was compared both with the spectrum of protonated TBD (in COC₁₃) and with that of the salt. The TBD base shows a septet signal at 1.58 ppm for the 3,7-methylene groups and two triplets at 2.64 and 3.23 ppm pertinent to the N-CH₂ groups. The latter signal was tentatively assigned to the 2,8-methylenes. On protonation the spectra are significantly changed, the most characteristic change is that the N-CH₂ signals merge to one triplet. In the C-₁₃ spectra all methylene signals suffer significant upfiled shifts. Spectra of TBD base cannot be recorded in chloroform due to rapid deprotonation of the solvent which gives rise to a spectrum for the salt.

Both the proton and carbon-13 spectrum of the salt show the changes diagnostic for salt formation, i.e. the merger of the N-CH₂ signals and the upfield shifts of the carbon signals. In the latter, apart from the signals of the benzoate part, three, and only three sharp signals appear for the methylene carbons indicating a structure symmetrical in respect of the two rings. The signal at 151.5 ppm was assigned to the quaternary carbon.

### 5) Fine structure

In analogy to the well known chemistry of guanidinium salts the positive charge generated by transfer of a proton from the acid to TBD base cannot be localized at a specific nitrogen atom, but is equally distributed over all three nitrogen atoms or, by another formalism it may be placed onto the bridgehead carbon atom:

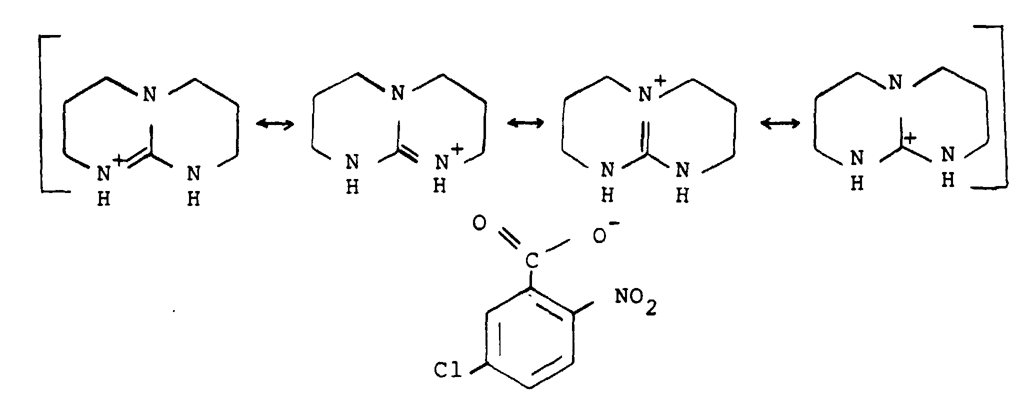

### TBD 4-chloro-3-nitrobenzoate (Example 12)

On evaporation of the acetone solution the product crystallized. After trituration with ether the crystals were filtered off. Yield of the crude product 1.70 g (100%) m.p. 148-158°C.

### TBD 5-nitrosalicylate (2-hydroxy-5-nitrobenzoate) (Example 13)

The crude product precipitated directly from the acetone solution, yield 1.46 g (90%).

### TBD 3-hydroxy-4-nitrobenzoate (Example 14)

The crude product precipitated directly from the acetone solution, yield 1.41 g (87%), m.p. 172-176°C.

### TBD 2-iodobenzoate (Example 15)

After evaporation for the acetone solution the residue was treated with ether giving 1.85 g (95%) of the crude product, m.p. 122-138°C.

### TBD 5-amino-5-hydroxybenzoate (Example 16)

When to a suspension of the acid in 10 ml of methanol TBD dissolved in 2.5 ml of methanol was added a violet solution formed. On evaporation and trituration with ether brown crystals (1.4 g, 96%) were obtained which melted between 172-182°C. After recrystallization from ethanol a product with an undefined m.p. was obtained suggesting that decomposition occurred during this operation.

### TBD 5-nitroisophthalate (5-nitrobenzene-1,3-dicarboxylic acid bis-TBD salt (Example 17)

On combination of the acetone solutions of the acid with a solution of four equivalents of TBD in acetone the product (1.34 g, 100%) precipitated immediately as white crystals of undefined m.p. remained undefined (212-260°C). Composition of the salt was also checked by ¹H-NHR.

### Examples 18-35: TBD salts of amino acids

The general procedure followed was to add to a suspension of the amino acid in ethanol a solution of TBD in the same solvent. On addition of TBD the amino acids went always into solution, indicating the formation of a salt, but the pH remained alkaline. This and the failure to obtain crystalline salts with almost all of the amino acids tried can be explained by the fact that due to internal salt formation with the amino group, amino acids are weak acids. A total of 20 different amino acids were tried of which only the following three gave crystallisable TBD salt i.e.: glycine (Example 18), L-glutamine (Example 19), and L-histidine (Example 20).

All the rest gave on evaporation of the ethanolic solutions syrups. Among them the syrups obtained from
Example 21: L-asparagin.
Example 22: L-valine.
Example 23: L-leucine.
Example 24: 4-aminobutyric acid (GABA).
crystallized on prolonged standing, but on treatment with a solvent they again dissolved.

The amino acids giving non-crystallizing syrups were as follows:
Example 25: L-glutamic acid.
Example 26: L-serine.
Example 27: L-lysine.
Example 28: L-proline.
Example 29: L-alanine.
Example 30: L-cysteine.
Example 31: L-4-hydroxyvaline.
Example 32: L-aspartic acid.
Example 33: L-phenylalanine.
Example 34: L-tryptophane.
Example 35: L-methionine.

Details of the preparation of the crystalline salts follow:

### TBD glycinate (Example 18)

To a suspension of glycine (0.75 g, 10 mmol) in ethanol (10 ml) a solution of TBD (1.25 g, 9 mmol) in ethanol (5 ml) was added. The clear solution obtained was evaporated and the crystalline residue dissolved in ethyl acetate (5 ml). The product crystallized in large prisms. Yield 0.72 g (38%), m.p. 146-148°C.

### TBD L-glutaminate (Example 19)

To a suspension of L-glutamine (0.73 g, 5 mmol) in ethanol (10 ml) TBD (0.63 g, 4 mmol) was added whereupon almost all of the glutamine went into solution. The filtered solution was evaporated and the crystalline residue triturated with ethyl acetate to give the product (0.70 g, 61%), m.p. 186-188°C (with decomposition).

### TBD L-histidinate (Example 20)

To a suspension of L-histidine (0.78 g, 5 mmol) in methanol (5 ml) TBD (0.68 g, 5 mmol) was added, whereupon almost all of histidine went into solution. The filtered solution was evaporated and the crystalline residue triturated with ether to give the product (1.15 g, 78%), m.p. 98-100°C (with decomposition).

The above examples are for reference and it will be appreciated that changes can be made while remaining within the scope of the invention.

### Pharmaceutical Activity

100 heterosexuals of either sex with frequent recurrence of genital herpes were topically treated in a double blind comparative study with either a composition (Composition A) consisting of the compound believed to have the formula (II) in 1% cream form, or a composition (Composition B) consisting of a known compound of the formula (IX) below (ACYCLOVIR) in 5% cream form:

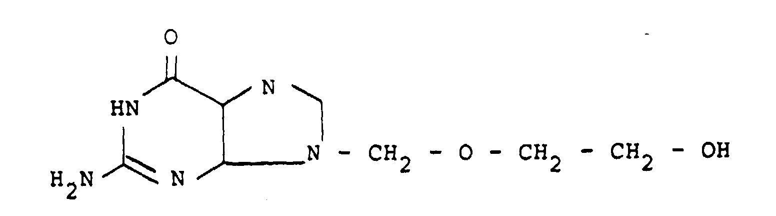

The treatment was in all cases started within 24 hours after the first symptoms or signs of a recurrence. The creams were applied to the lesions four times a day for five days. Before treatment started, on days 1,2 and 4, a culture for herpes virus was carried out. Reexaminations were carried out on those days and if needed on days 7, 10 and 14. The major factor used for assessment of efficacy was the time to complete healing of all lesions. Duration of pruritus, pain and viral shedding were also recorded. In the group of patients treated with Composition A, the time to complete healing was reduced from 7.5 to 3.4 days, which was not considered to be a significant reduction. The mean duration of pain was 1.4 days in the Composition A group and 2.8 days in the Composition B group (p<0.01). The mean duration of pruritus and viral shedding was about the same in both groups. It was concluded that the efficiency of topical application of Composition A may be due to combined antiviral and immunomodulatory activities. Recent studies have shown that the compound appears to be effective as a result of activity on the surface of the virus.

## Claims (Claims for the following Contracting State(s): AT, BE, CH, LI, DE, DK, FR, GB, IT, LU, NL, SE)

1. A pharmaceutically active compound prepared from 1,3,4,6,7,8-hexahydro-2H-pyrimido-(1,2-a)-pyrimidine (HHPP), said compound having the formula 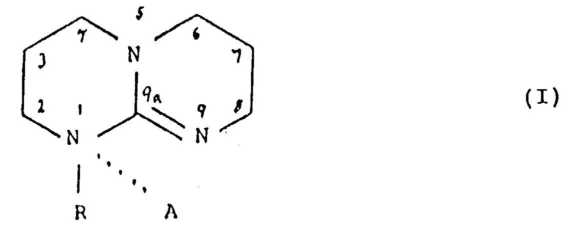 wherein R is hydrogen or an alkyl group and wherein the dotted line represents a salt linkage or, when R is hydrogen, such hydrogen being replaced thus that a covalent bond is formed between 1,3,4,6,7,8-hexahydro-2H-pyrimido-(1,2-a)-pyrimidine and the group A, and wherein A is a residue of an acid or an acid anhydride selected from indole carboxylic acid, 2,4,6-trihydroxy benzoic acid, 3-nitro-o-toluic acid, phthalic anhydride and benzoic anhydride, or an ester or acid chloride thereof; the acid being further selected from 4-chloro-2-nitrobenzoic acid, 4-nitrophenylacetic acid, 2,4-dinitrophenylacetic acid, 5-chloro-2-nitrobenzoic acid, 4-chloro-3-nitrobenzoic acid, 5-nitrosalicylic acid, (2-hydroxy-5-nitrobenzoic acid), 3-hydroxy-4-nitrobenzoic acid, 2-iodobenzoic acid, 5-amino-5-hydroxybenzoic acid, 5-nitroisophthalic acid, (5-nitrobenzene-1,3-dicarboxylic acid), glycine, L-glutamine, L-histidine, L-valine, L-leucine, 4-aminobutyric acid (GABA), L-glutamic acid, L-serine, L-lysine, L-proline, L-alanine, L-cysteine, L-4-hydroxyvaline, L-aspartic acid, L-phenylalanine, L-tryptophane and L-methionine and boric acid; or a neutral amine salt of such a pharmaceutically active compound.

2. A pharmaceutically active compound as claimed in claim 1, which is a salt formed between the 1-amino group of the compound of the formula (I) and a carboxyl group derived from the acid or acid anhydride.

3. A pharmaceutically active compound according to claim 1 or 2 wherein the acid or anhydride is selected from:
indole-2-carboxylic acid,
2,4,6-trihydroxy-benzoic acid,
3-nitro-o-toluic acid,
benzoic anhydride,
4-chloro-2-nitrobenzoic acid,
4-nitrophenylacetic acid,
2,4-dinitrophenylacetic acid,
5-chloro-2-nitrobenzoic acid,
4-chloro-3-nitrobenzoic acid,
5-nitrosalicylic acid, (2-hydroxy-5-nitrobenzoic acid,)
3-hydroxy-4-nitrobenzoic acid,
2-iodobenzoic acid,
5-amino-5-hydroxybenzoic acid,
5-nitroisophthalic acid (5-nitrobenzene-1,3-dicarboxylic acid),
glycine,
L-glutamine,
and L-histidine;
or a neutral salt thereof.

4. A pharmaceutical preparation comprising a pharmaceutically active compound as claimed in any one of claims 1 to 3, in a pharmaceutically acceptable carrier.

5. A pharmaceutical preparation as claimed in claim 4, wherein the carrier is one which allows topical application.

6. A pharmaceutical composition as claimed in claim 5, wherein the carrier is in the form of a cream.

7. The use of a compound as claimed in any one of claims 1 to 3, in the manufacture of a medicament for the treatment of viral diseases.

8. The use of a compound as claimed in any one of claims 1 to 3, in the manufacture of a medicament for the treatment of herpes or human papilloma virus.

## Claims (Claims for the following Contracting State(s): ES, GR)

1. A method of preparing a pharmaceutically active compound of the formula 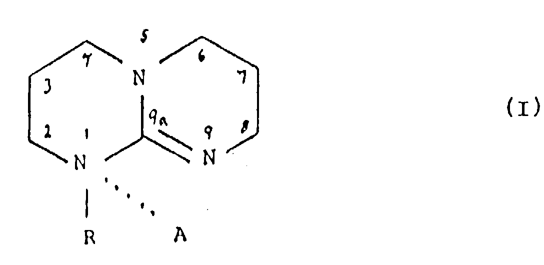 wherein R is hydrogen or an alkyl group and wherein the dotted line represents a salt linkage or, when R is hydrogen, such hydrogen being replaced thus that a covalent bond is formed between 1,3,4,6,7,8-hexahydro-2H-pyrimido-(1,2-a)-pyrimidine and the group A, and wherein A is a residue of an acid or an acid anhydride selected from indole carboxylic acid, 2,4,6-trihydroxy benzoic acid, 3-nitro-o-toluic acid, phtalic anhydride and benzoic anhydride, or an ester or acid chloride thereof; the acid being further selected from 4-chloro-2-nitrobenzoic acid, 4-nitrophenylacetic acid, 2,4-dinitrophenylacetic acid, 5-chloro-2-nitrobenzoic acid, 4-chloro-3-nitrobenzoic acid, 5-nitrosalicylic acid, (2-hydroxy-5-nitrobenzoic acid), 3-hydroxy-4-nitrobenzoic acid, 2-iodobenzoic acid, 5-amino-5-hydroxybenzoic acid, 5-nitroisophthalic acid, (5-nitrobenzene-1,3-dicarboxylic acid), glycine, L-glutamine, L-histidine, L-valine, L-leucine, 4-aminobutyric acid (GABA), L-glutamic acid, L-serine, L-lysine, L-proline, L-alanine, L-cysteine, L-4-hydroxyvaline, L-aspartic acid, L-phenylalanine, L-tryptophane and L-methionine and boric acid; or a neutral amine salt of such a pharmaceutically active compound, comprising reacting 1,3,4, 6,7,8-hexahydro-2H-pyrimido-(1,2-a)-pyrimidine (HHPP) with one of said acids or acid anhydrides, and optionally neutralising the resultant product.

2. A method as claimed in claim 1, wherein the dotted line represents a salt linkage between the 1-amino group of the compound of the formula (I) and a carboxyl group derived from the acid or acid anhydride.

3. A method as claimed in claim 1 or 2 wherein the acid or acid anhydride is selected from:
indole-2-carboxylic acid,
2,4,6-trihydroxy-benzoic acid,
3-nitro-o-toluic acid,
benzoic anhydride,
4-chloro-2-nitrobenzoic acid,
4-nitrophenylacetic acid,
2,4-dinitrophenylacetic acid,
5-chloro-2-nitrobenzoic acid,
4-chloro-3-nitrobenzoic acid,
5-nitrosalicylic acid, (2-hydroxy-5-nitrobenzoic acid,)
3-hydroxy-4-nitrobenzoic acid,
2-iodobenzoic acid,
5-amino-5-hydroxybenzoic acid,
5-nitroisophthalic acid (5-nitrobenzene-1,3-dicarboxylic acid),
glycine,
L-glutamine,
and L-histidine.

4. A method as claimed in any of claims 1 to 3, further comprising incorporating the pharmaceutically active compound in a pharmaceutically acceptable carrier.

5. A method as claimed in claim 4, wherein the carrier is one which allows topical application.

6. A method as claimed in claim 5, wherein the carrier is in the form of a cream.

7. The use of a compound as prepared in any one of claims 1 to 3, in the manufacture of a medicament for the treatment of viral diseases.

8. The use of a compound as prepared in any one of claims 1 to 3, in the manufacture of a medicament for the treatment of herpes or human papilloma virus.

## Patentansprüche (Patentansprüche für folgende(n) Vertragsstaat(en): AT, BE, CH, LI, DE, DK, FR, GB, IT, LU, NL, SE)

1. Pharmazeutisch aktive Verbindung, dargestellt aus 1,3,4,6,7,8-Hexahydro-2H-pyrimido-(1,2-a)-pyrimidin (HHPP), wobei besagte Verbindung die Formel 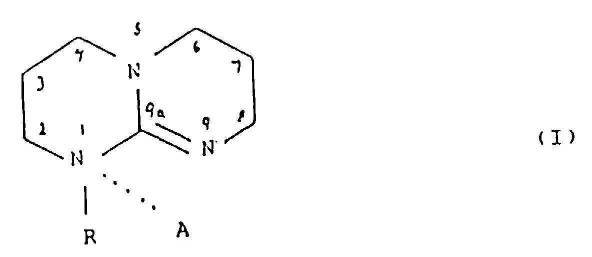 besitzt, worin R Wasserstoff oder eine Alkylgruppe ist und die punktierte Linie eine Salzbindung darstellt, oder wenn R Wasserstoff ist, dieser Wasserstoff derart ersetzt wird, dass zwischen dem 1,3,4,6,7,8-Hexahydro-2H-pyrimido-(1,2-a)-pyrimidin und der Gruppe A eine kovalente Bindung gebildet wird, und worin A ein Rest einer Säure oder eines Säureanhydrids ist, welche Säure oder Säureanhydrid ausgewählt wird aus Indolcarbonsäure, 2,4,6-Trihydroxybenzoesäure, 3-Nitro-o-toluolsäure, Phthalsäureanhydrid und Benzoesäureanhydrid, oder deren Ester oder Säureschlorid, wobei die Säure ferner ausgewählt wird aus 4-Chloro-2-nitrobenzoesäure, 4-Nitrophenylessigsäure, 2,4-Dinitrophenylessigsäure, 5-Chloro-2-nitrobenzoesäure, 4-Chloro-3-nitrobenzoesäure, 5-Nitrosalicylsäure (2-Hydroxy-5-nitrobenzoesäure), 3-Hydroxy-4-nitrobenzoesäure, 2-Jodobenzoesäure, 5-Amino-5-hydroxybenzoesäure, 5-Nitroisophthalsäure (5-Nitrobenzen-1,3-dicarbonsäure), Glycin, L-Glutamin, L-Histidin, L-Valin, L-Leucin, 4-Aminobuttersäure (GABA), L-Glutaminsäure, L-Serin, L-Lysin, L-Prolin, L-Alanin, L-Cystein, L-4-Hydroxyvalin, L-Asparaginsäure, L-Phenylalanin, L-Tryptophan und L-Methionin und Borsäure; oder ein neutrales Aminsalz einer solchen pharmazeutisch aktiven Verbindung.

2. Pharmazeutisch aktive Verbindung nach Anspruch 1, die ein Salz darstellt, das zwischen der 1-Amino-Gruppe der Verbindung der Formel (I) und einer Carboxylgruppe gebildet wird, die von der Säure oder vom Säureanhydrid abgeleitet wird.

3. Pharmazeutisch aktive Verbindung nach Anspruch 1 und 2, worin die Säure oder das Anhydrid ausgewählt werden aus:
Indol-2-carbonsäure
2,4,6-Trihydroxybenzoesäure,
3-Nitro-o-toluolsäure,
Benzoesäureanhydrid,
4-Chloro-2-nitrobenzoesäure,
4-Nitrophenylessigsäure,
2,4-Dinitrophenylessigsäure,
5-Chloro-2-nitrobenzoesäure,
4-Chloro-3-nitrobenzoesäure,
5-Nitrosalicylsäure (2-Hydroxy-5-nitrobenzoesäure),
3-Hydroxy-4-nitrobenzoesäure,
2-Jodobenzoesäure,
5-Amino-5-hydroxybenzoesäure,
5-Nitroisophthalsäure (5-Nitrobenzene-1,3-dicarbonsäure),
Glycin,
L-Glutamin
und L-Histidin
oder deren neutralem Salz.

4. Pharmazeutisches Präparat, das eine pharmazeutisch aktive Verbindung nach einem der Ansprüche 1 bis 3 in einer pharmazeutisch zulässigen Trägersubstanz umfasst.

5. Pharmazeutisches Präparat nach Anspruch 4, worin die Trägersubstanz eine solche ist, die eine örtliche Aufbringung erlaubt.

6. Pharmazeutisches Präparat nach Anspruch 5, worin die Trägersubstanz in Form einer Creme vorliegt.

7. Verwendung einer Verbindung nach einem der Ansprüche 1 bis 3 zur Herstellung eines Medikamentes als Mittel zur Behandlung von viraler Erkrankungen.

8. Verwendung einer Verbindung nach einem der Ansprüche 1 bis 3 zur Herstellung eines Medikamentes als Mittel zur Behandlung von Herpes oder Human-Papillomavirus.

## Patentansprüche (Patentansprüche für folgende(n) Vertragsstaat(en): ES, GR)

1. Verfahren zur Herstellung einer Pharmazeutisch aktiven Verbindung der Formel 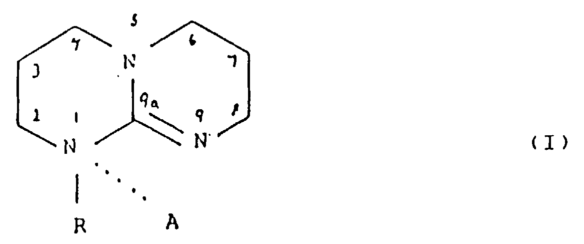 worin R Wasserstoff oder eine Alkylgruppe ist und die punktierte Linie eine Salzbindung darstellt, oder wenn R Wasserstoff ist, dieser Wasserstoff derart ersetzt wird, dass zwischen dem 1,3,4,6,7,8-Hexahydro-2H-pyrimido-(1,2-a)-pyrimidin und der Gruppe A eine kovalente Bindung gebildet wird, und worin A ein Rest einer Säure oder eines Säureanhydrids ist, welche Säure, oder Säureanhydrid ausgewählt wird aus Indolcarbonsäure, 2,4,6-Trihydroxybenzoesäure, 3-Nitro-o-toluolsäure, Phthalsäureanhydrid und Benzoesäureanhydrid, oder deren Ester oder Säurechlorid, wobei die Säure ferner ausgewählt wird aus 4-Chloro-2-nitrobenzoesäure, 4-Nitro-phenylessigsäure, 2,4-Dinitrophenylessigsäure, 5-Chloro-2-nitrobenzoesäure, 4-Chloro-3-nitrobenzoesäure, 5-Nitrosalicylsäure (2-Hydroxy-5-nitrobenzoesäure), 3-Hydroxy-4-nitrobenzoesäure, 2-Jodobenzoesäure, 5-Amino-5-hydroxybenzoesäure, 5-Nitroisophthalsäure (5-Nitrobenzen-1,3-dicarbonsäure), Glycin, L-Glutamin, L-Histidin, L-Valin, L-Leucin, 4-Aminobuttersäure (GABA), L-Glutaminsäure, L-Serin, L-Lysin, L-Prolin, L-Alanin, L-Cystein, L-4-Hydroxyvalin, L-Asparaginsäure, L-Phenylalanin, L-Tryptophan und L-Methionin und Borsäure; oder eines neutralen Aminsalzes einer solchen pharmazeutisch aktiven Verbindung umfassend die Umsetzung von 1, 3,4,6,7,8-Hexahydro-2H-pyrimido-(1,2-a)-pyrimidin (HHPP) mit einer bzw. einem der genannten Säuren oder Säureanhydride und gegebenenfalls die Neutralisation des resultierenden Produkts.

2. Verfahren nach Anspruch 1, bei welchem die punktierte Linie eine Salzbindung zwischen der 1-Amino-Gruppe der Verbindung der Formel (I) under einer Carboxylgruppe darstellt, die von der Säure oder vom Säureanhydrid abgeleitet wird.

3. Verfahren nach Anspruch 1 oder 2, bei welchem die Säure oder das Anhydrid ausgewählt werden aus:
Indol-2-carbonsäure
2,4,6-Trihydroxybenzoesäure,
3-Nitro-o-toluolsäure,
Benzoesäureanhydrid,
4-Chloro-2-nitrobenzoesäure,
4-Nitrophenylessigsäure,
2,4-Dinitrophenylessigsäure,
5-Chloro-2-nitrobenzoesäure,
4-Chloro-3-nitrobenzoesäure,
5-Nitrosalicylsäure (2-Hydroxy-5-nitrobenzoesäure),
3-Hydroxy-4-nitrobenzoesäure,
2-Jodobenzoesäure,
5-Amino-5-hydroxybenzoesäure,
5-Nitroisophthalsäure (5-Nitrobenzene-1,3-dicarbonsäure),
Glycin,
L-Glutamin
und L-Histidin

4. Verfahren nach einem der Ansprüche 1 bis 3, weiter umfassend das Einverleiben der pharmazeutisch aktiven Verbindung in einer pharmazeutisch zulässigen Trägersubstans.

5. Verfahren nach Anspruch 4, bei welchem die Trägersubstanz eine solche ist, die eine örtliche Aufbringung erlaubt.

6. Verfahren nach Anspruch 5, bei welchem die Trägersubstanz in Form einer Creme vorliegt.

7. Verwendung einer Verbindung, die nach einem der Ansprüche 1 bis 3 hergestellt ist, zur Herstellung eines Medikamentes zur Behandlung viraler Erkrankungen.

8. Verwendung einer Verbindung, die nach einem der Ansprüche 1 bis 3 hergestellt ist, zur Herstellung eines Medikamentes zur Behandlung von Herpes oder Human-Papillomavirus.

## Revendications (Revendications pour l'(les) Etat(s) contractant(s) suivant(s): AT, BE, CH, LI, DE, DK, FR, GB, IT, LU, NL, SE)

1. Composé pharmaceutiquement actif préparé à partir de 1,3,4,6,7,8-hexahydro-2H-pyrimido-(1,2-a)-pyrimidin (HHPP) ce composé ayant pour formule: 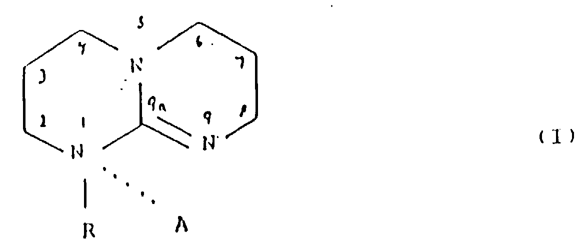 da ligne en pointillé une liaison sel ou, lorsque R est de l'hydrogène, cet hydrogène est remplacé en sorte qu'une liaison covalente soit formée entre la 1,3,4,6,7,8-hexahydro-2H-pyrimido-(1,2-a)-pyrimidine et le groupe A, A est un résidu d'un acide ou d'un anhydride d'acide choisi parmi l'acide indole carboxylique, l'acide 2,4,6-trihydroxybenzoïque, l'acide 3-nitro-o-toluique, l'anhydride phtalique et l'anhydride benzoïque, ou un ester ou un chlorure d'acide de ces produits; l'acide étant choisi en outre parmi l'acide 4-chloro-2-nitrobenzoïque, l'acide 4-nitrophényl acétique, l'acide 2,4-dinitrophénylacétique, l'acide 5-chloro-2-nitrobenzoïque, l'acide 4-chloro-3-nitrobenzoïque, l'acide 5-nitrosalicylique (acide 2-hydroxy-5-nitrobenzoïque), l'acide 3-hydroxy-4-nitrobenzoïque, l'acide 2-iodobenzoïque, l'acide 5-amino-5-hydroxybenzoïque, l'acide 5-nitroisophtalique, (l'acide 5-nitrobenzène-1,3-dicarboxylique), la glycine, la L-glutamine, 1 a L-histidine, L-valine, la L-leucine, l'acide 4-aminobutyrique (GABA), l'acide L-glutamique, la L-sérine, la L-lysine, la L-proline, la L-alanine, la L-cystéine, la L-4-hydroxyvaline, l'acide L-aspartique, la L-phénylalanine, le L-tryptophane, la L-methinionine et l'acide borique; ou un sel aminé neutre d'un tel composé pharmaceutiquement actif.

2. Composé pharmaceutiquement actif selon la revendication 1, qui est un sel formé entre le groupe amino 1 du composé de formule (I) et un groupe carboxyle dérivé d'acide ou d'anhydride d'acide.

3. Composé pharmaceutiquement actif selon la revendication 1 ou 2, dans lequel l'acide ou l'anhydride est choisi parmi les substances suivantes:
acide indole-2-carboxylique
acide 2,4,6-trihydroxy-benzoïque
acide 3-nitro-o-toluique
anhydride benzoïque
acide 4-chloro-2-nitrobenzoïque
acide 4-nitrophénylacétique
acide 2,4-dinitrophénylacétique
acide 5-chloro-2-nitrobenzoïque
acide 4-chloro-3-nitrobenzoïque
acide 5-nitrosalicylique (acide 2-hydroxy-5-nitrobenzoïque)
acide 3-hydroxy-4-nitrobenzoïque
acide 2-iodobenzoïque
acide 5-amino-5-hydroxybenzoïque
acide 5-nitroisophtalique (acide 5-nitrobenzène-1,3-dicarboxylique)
glycine
L-glutamine, et
L-histidine
ou un sel neutres de ces substances.

4. Préparation pharmaceutique comprenant un composé pharmaceutiquement actif selon l'une quelconque des revendications 1 à 5 dans un véhicule pharmaceutiquement acceptale.

5. Préparation pharmaceutique selon la revendication 4, dans laquelle le véhicule est un véhicule qui permet une application topique.

6. Composition pharmaceutique selon la revendication 5, dans laquelle le véhicule se présente sous la forme d'une crème.

7. Emploi d'un composé selon l'une quelconque des revendications 1 à 3 pour la fabrication d'un médicament permettant le traitement d'affections virales.

8. Emploi d'un composé selon l'une quelconque des revendications 1 à 3 pour la fabrication d'un médicament permettant le traitement de l'herpès ou du virus papillome humain.

## Revendications (Revendications pour l'(les) Etat(s) contractant(s) suivant(s): ES, GR)

1. Procédé de préparation d'un composé pharmaceutiquement actif ayant pour formule: 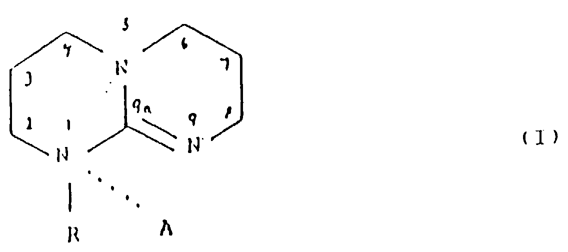 dans laquelle R est de l'hydrogène ou un groupe alkyle, la ligne en pointillé est une liaison sel ou, lorsque R est de l'hydrogène, cet hydrogène est remplacé en sorte qu'une liaison covalente soit formée entre la 1,3,4,6,7,8-hexahydro-2H-pyrimido-(1,2-a)-pyrimidine et le groupe A, A est un résidu d'un acide ou d'un anhydride d'acide choisi parmi l'acide indole carboxylique, l'acide 2,4,6-trihydroxybenzoïque, l'acide 3-nitro-o-toluique, l'anhydride phtalique et l'anhydride benzoïque, ou un ester ou un chlorure d'acide de ces produits; l'acide étant choisi en outre parmi l'acide 4-chlore-2-nitrobenzoïque, l'acide 4-nitrophénylacétique, l'acide 2,4-dinitrophénylacétique, l'acide 5-chlore-2-nitrobenzoïque, l'acide 4-chloro-3-nitrobenzoïque, l'acide 5-nitrosalicylique (acide 2-hydroxy-5-nitrobenzoique), l'acide 3-hydroxy-4-nitrobenzoïque, l'acide 2-iodobenzoïque, l'acide 5-amino-5-hydroxybenzoïque, l'acide 5-nitroisophtalique, (l'acide 5-nitrobenzène-1,3-dicarboxylique); la glycine, la L-glutamine, la L-histidine, la L-valine, la L-leucine, l'acide 4-aminobutyrique (GABA), l'acide L-glutamique, la L-sérine, la L-lysine, la L-proline, la L-alanine, la L-cystéine, la L-4-hydroxyvaline, l'acide L-aspartique,la L-phénylalanine, le L-tryptophane, la L-méthionine et l'acide borique; ou un sel aminé neutre d'un tel composé pharmaceutiquement actif, consistant à faire réagir la 1,3,4,6,7,8-hexahydro-2H-pyrimido-(1,2-a)-pyrimidine (HHPP) avec l'un des acides ou des anhydrides d'acides précités et, éventuellement, à neutraliser le produit obtenu.

2. Procédé selon la revendication 1, dans lequel la ligne en pointillé est une liasion sel formée entre le groupe amino 1 du composé de formule (I) et un groupe carboxyle dérivé d'acide ou d'anhydride d'acide.

3. Procédé selon la revendication 1 ou 2 dans lequel l'acide ou l'anhydride d'acide est choisi parmi les substances suivantes:
acide indole-2-carboxylique
acide 2,4,6-trihydroxy-benzoïque
acide 3-nitro-o-toluique
anhydride benzoïque
acide 4-chloro-2-nitrobenzoïque
acide 4-nitrophénylacétique
acide 2,4-dinitrophénylacétique
acide 5-chloro-2-nitrobenzoïque
acide 4-chloro-3-nitrobenzoïque
acide 5-nitrosalicylique (acide 2-hydroxy-5-nitrobenzoïque)
acide 3-hydroxy-4-nitrobenzoïque
acide 2-iodobenzoïque
acide 5-amino-5-hydroxybenzoïque
acide 5-nitroisophtalique (acide 5-nitrobenzène-1,3-dicarboxylique)
glycine
L-glutamine, et
L-histidine
ou un sel neutres de ces substances.

4. Procédé selon l'une quelconque des revendications 1 à 3, de plus consistant à incorporer le composé pharmaceutiquement actif dans un véhicule pharmaceutiquement acceptable.

5. Procédé selon la revendication 4, dans laquelle le véhicule est un véhicule qui permet une application topique.

6. Procédé selon la revendication 5, dans laquelle le véhicule se présente sous la forme d'une crème.

7. Emploi d'un composé préparé par un procédé selon l'une quelconque des revendications 1 à 3 pour la fabrication d'un médicament permettant le traitement d'affections virales.

8. Emploi d'un composé préparé par un procédé selon l'une quelconque des revendications 1 à 3, pour la fabrication d'un médicament permettant le traitement de l'herpès ou du virus papillome humain.
